(19) Europäisches Patentamt
European Patent Office
Office européen des brevets

(11) **EP 1 235 592 B1**

(12) **EUROPÄISCHE PATENTSCHRIFT**

(45) Veröffentlichungstag und Bekanntmachung des Hinweises auf die Patenterteilung:
**14.11.2007 Patentblatt 2007/46**

(51) Int Cl.:
*A61K 39/395* *(2006.01)*   *A61P 9/10* *(2006.01)*
*A61P 25/28* *(2006.01)*

(21) Anmeldenummer: **00993306.0**

(22) Anmeldetag: **06.12.2000**

(86) Internationale Anmeldenummer:
**PCT/DE2000/004371**

(87) Internationale Veröffentlichungsnummer:
**WO 2001/041803 (14.06.2001 Gazette 2001/24)**

(54) **KOMBINATION VON VERBINDUNGEN, WELCHE DIE BIOLOGISCHEN WIRKUNGEN VON TNF-ALPHA UND CD95L HEMMEN, IN EINEM ARZNEIMITTEL**

COMBINATION OF COMPOUNDS THAT INHIBIT THE BIOLOGICAL EFFECTS OF TNF-ALPHA AND CD95L IN A MEDICAMENT

COMBINAISON DE COMPOSES INHIBANT LES EFFETS BIOLOGIQUES DE TNF-ALPHA ET DE CD95L DANS UN MEDICAMENT

(84) Benannte Vertragsstaaten:
**AT BE CH CY DE DK ES FI FR GB GR IE IT LI LU MC NL PT SE TR**

(30) Priorität: **07.12.1999 DE 19959049**

(43) Veröffentlichungstag der Anmeldung:
**04.09.2002 Patentblatt 2002/36**

(73) Patentinhaber: **Deutsches Krebsforschungszentrum Stiftung des öffentlichen Rechts 69120 Heidelberg (DE)**

(72) Erfinder:
 • **KRAMMER, Peter**
 **69120 Heidelberg (DE)**
 • **MARTIN-VILLALBA, Ana**
 **69117 Heidelberg (DE)**

(74) Vertreter: **Weiss, Wolfgang et al Weickmann & Weickmann Patentanwälte Postfach 86 08 20 81635 München (DE)**

(56) Entgegenhaltungen:
 • **NAKAZAWA T ET AL: "Cytolytic mechanisms involved in non-MHC-restricted cytotoxicity in Chediak-Higashi syndrome." CLINICAL AND EXPERIMENTAL IMMUNOLOGY, (1999 OCT) 118 (1) 108-14. , XP000984560**
 • **HATTORI K ET AL: "A metalloproteinase inhibitor prevents lethal acute graft-versus-host disease in mice." BLOOD, (1997 JUL 15) 90 (2) 542-8. , XP000984491**
 • **MARTIN-VILLALBA A ET AL: "CD95 LIGAND (FAS-L/APO-1L) AND TUMOR NECROSIS FACTOR-RELATED APOPTOSIS-INDUCING LIGAND MEDIATE ISCHEMIA-INDUCED APOPTOSIS IN NEURONS" JOURNAL OF NEUROSCIENCE, NEW YORK, NY, US, Bd. 19, Nr. 10, 15. Mai 1999 (1999-05-15), Seiten 3809-3817, XP000980204 ISSN: 0270-6474**
 • **MARTIN-VILLALBA, A. (1) ET AL: "Protection against brain ischemia induced apoptosis mediated by the CD95 ligand system in lpr mice." SOCIETY FOR NEUROSCIENCE ABSTRACTS, (1998) VOL. 24, NO. 1-2, PP. 275. MEETING INFO.: 28TH ANNUAL MEETING OF THE SOCIETY FOR NEUROSCIENCE, PART 1 LOS ANGELES, CALIFORNIA, USA NOVEMBER 7-12, 1998 SOCIETY FOR NEUROSCIENCE. , XP000984977**
 • **CROW, MICHAEL T. ET AL: "The role of the tumor suppressor gene p53 in cardiomyocyte apoptosis" HEART FAILURE REV. (1998), 3(1), 45-61 , XP000984631**
 • **JEREMIAS I ET AL: "Involvement of CD95/Apo1/Fas in cell death after myocardial ischemia." CIRCULATION, (2000 AUG 22) 102 (8) 915-20. , XP000984492**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 235 592 B1

- L. ALEXOPOULOU ET AL: "A murine transmembrane tumor necrosis factor (TNF) transgene induces arthritis by cooperative p55/p75 TNF receptor signaling" EUR. J. IMMUNOL., Bd. 27, 1997, Seiten 2855-2592,
- WIM VAN DEN BERG ET AL: "Role of tumour necrosis factor alpha in experimental arthritis : separate activity of interleukin 1 beta in chronicity and cartilage destruction" ANN RHEUM DIS, Bd. 58, 1999, Seiten I40-I48,
- P. SCHNEIDER ET AL: "Conversion of Membrane-bound Fas(CD95) Ligand to its soluble form is associated with Downregulation of its proapptoti activity and loss of liver toxicity" J. EXP. MED., Bd. 187, Nr. 8, 8. April 1998 (1998-04-08), Seiten 1205-1213,

## Beschreibung

Die vorliegende Erfindung betrifft ein Arzneimittel, das eine oder mehrere Verbindungen gemäß den Patentansprüchen enthält, welche die biologischen

Wirkungen von TNF-$\alpha$ und CD95L hemmen, beispielsweise dadurch, daß die Bindung dieser Liganden an ihre Rezeptoren blockiert wird und damit eine Signaltransduktion unterbleibt. Vorzugsweise handelt es sich bei diesen Verbindungen um einen neutralisierenden anti-TNF-$\alpha$-Antikörper und einen neutralisierenden anti-CD95L-Antikörper. Die vorliegende Erfindung betrifft auch die Verwendung der vorstehenden Verbindungen zur Prävention oder Behandlung eines Schlaganfalls oder Herzinfarkts.

Der Tod durch Schlaganfall stellt die dritthäufigste Todesursache in den meisten westlichen Staaten dar. Darüber hinaus stehen die durch Schlaganfälle hervorgerufenen Behinderungen, beispielsweise Lähmungen, praktisch an erster Stelle unter den monokausalen Behinderungen. Die Behandlung des Schlaganfalls bestand bisher hauptsächlich in der unterstützenden Pflege und der Verhinderung von weiteren, dadurch bedingten Komplikationen. Die gegenwärtigen experimentellen und klinischen Daten deuten darauf hin, daß das therapeutische Fenster variabel ist und auch 6 bis 8 Stunden übersteigen kann. Dieses variable Intervall wird durch die ischämische Penumbra bestimmt. Dabei handelt es sich um einen Bereich, der das Zentrum der ischämischen Läsion umgibt und bis zu dessen Absterben Stunden vergehen können. Inzwischen häufen sich die Anzeichen dafür, daß die Neuronen in der ischämischen Penumbra einer Apoptose unterliegen. Somit sind die Therapien am meisten erfolgsversprechend, die auf einer neuroprotektiven Strategie basieren, d.h. auf eine Unterdrückung der Apoptose hinzielen.

Zwei Mitglieder der Tumornekrosefaktor(TNF)-Familie, CD95 (auch Fas oder ApoI genannt) und der TNF-Rezeptor 1 (TNF-R1, auch p55 oder CD120a genannt), sind häufig an der Auslösung von Apoptose beteiligt. Diese Rezeptoren zeichnen sich durch eine homologe zytoplasmatische Aminosäuresequenz aus (die "Todes-Domäne"), die für die Transduktion des apoptotischen Signals von ausschlaggebender Bedeutung ist. Bei den natürlichen Liganden CD95L bzw. TNF handelt es sich um strukturell verwandte Transmembranproteine vom Typ II. Die Bindung des trimerisierten Liganden an den Rezeptor führt zur Rekrutierung des Proteins FADD ("Fass-associated death domain", auch MORT1 genannt). Nach Rekrutierung von FADD wird Caspase-8 durch Selbstspaltung aktiviert und schließlich durchlaufen die Zellen durch Aktivierung weiter abwärts liegender Effektor-Caspasen eine Apoptose.

Im Anschluß an eine Ischämie im Gehirn wird in der ischämischen Penumbra die Expression von TNF, CD95L und CD95 gesteigert. Die Rolle von TNF bei mit Gehirn-Ischämie in Zusammenhang stehenden Schädigungen ist dabei aber umstritten, jedenfalls resultierte die Verabreichung von TNF vor dem ischämischen Infarkt in einer signifikant verringerten Infarktgröße. Die Rolle von CD95/CD95L scheint eher eindeutig schädlich zu sein. Allerdings war bisher vollkommen unklar, ob diese beiden Liganden/Rezeptor-Systeme bei der Induktion der Ischämie kooperieren oder gegeneinander arbeiten. Jedenfalls gibt es bisher keine zufriedenstellende Therapie, um bei gefährdeten Personen einer Ischämie im Gehirn bzw. einem Schlaganfall vorbeugen zu können oder zumindest die dabei auftretenden Schädigungen verhindern oder wenigstens reduzieren zu können.

In dem Dokument Clinical and Experimental Immunology, Oct. 1999, 118 (1), 108 - 114 werden anti-FasL monoklonale Antikörper, anti-TNF-alpha polyklonale Antikörper oder eine Mischung beider Antikörper, die einem Cytotoxizitätstest beigemengt werden, beschrieben. Neben diesen genannten Antagonisten von FasL und TNF-alpha, sind im Medium des Zytotoxizitätstests auch noch Lymphozyten von Patienten sowie $^{51}$Cr enthalten.

Ein in der Medizin zur Prävention oder Therapie einsetzbares Arzneimittel wird mit dieser Zusammensetzung nicht offenbart.

Das Dokument Blood, 1997, 90(2), 542 - 548 offenbart eine Zusammensetzung, die einen Metallproteinase-Inhibitor (KB-R7785) enthält, der gleichzeitig die Freisetzung von TNF-alpha und CD95L hemmt.

Jedoch ist der durch die Substanz KB-R7785 erzielte Effekt, nämlich "Hemmung der Freisetzung" von TNF-alpha und CD95L nicht mit einer Beeinflussung bzw. Hemmung der biologischen Wirkung gleichzusetzen. Sie wird auch nicht als Antagonist von TNF-alpha und CD95L offenbart. Daher wird kein Arzneimittel offenbart, das Verbindungen enthält, die die biologische Wirkung von TNF-alpha und CD95L hemmen.

In dem Dokument The Journal of Neuroscience, May 1999, 19(10), 3809 - 3817 ist beschrieben, dass das Medikament FK506 die Expression von "death-inducing ligands" (DILs) verhindert. Die Verhinderung von Schlaganfall wird ebenfalls erwähnt.

Jedoch offenbart dieses Dokument nicht, dass FK605 in der Lage ist, die biologische Wirkung zu hemmen. Da lediglich in allgemeiner Form die Expression von DILs, die nicht nur TNF-alpha und CD95L umfassen, erwähnt wird, wird daher kein Antagonist beschrieben.

Somit liegt der vorliegenden Erfindung das technische Problem zugrunde, Mittel bereitzustellen, mit denen - beispielsweise bei gefährdeten Personen - einer Ischämie im Gehirn bzw. einem Schlaganfall vorgebeugt werden kann und/oder die dabei auftretenden Schädigungen verhindert oder reduziert werden können.

Die Lösung dieses technischen Problems erfolgt durch die Bereitstellung der in den Patentansprüchen gekennzeichneten Ausführungsformen.

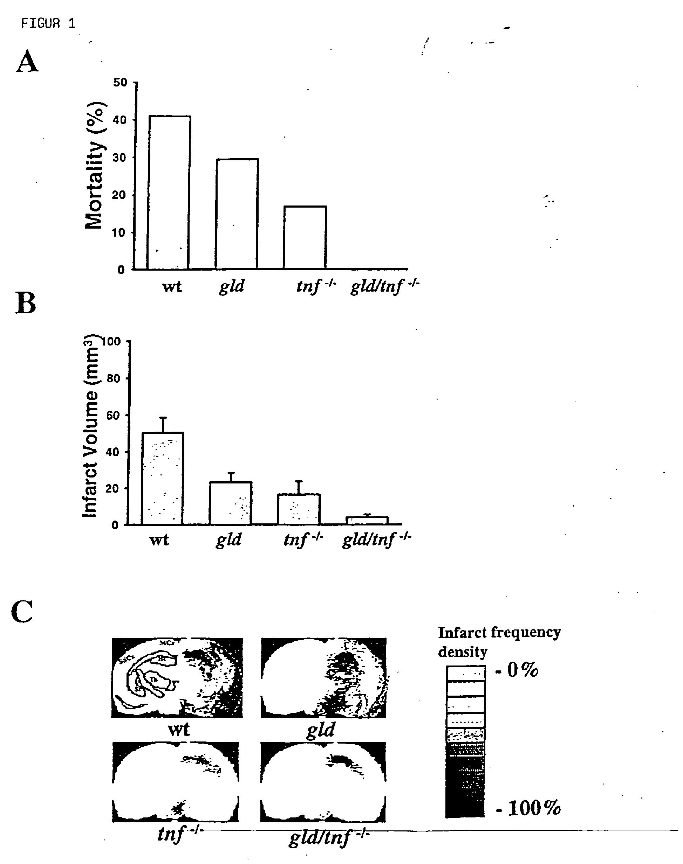

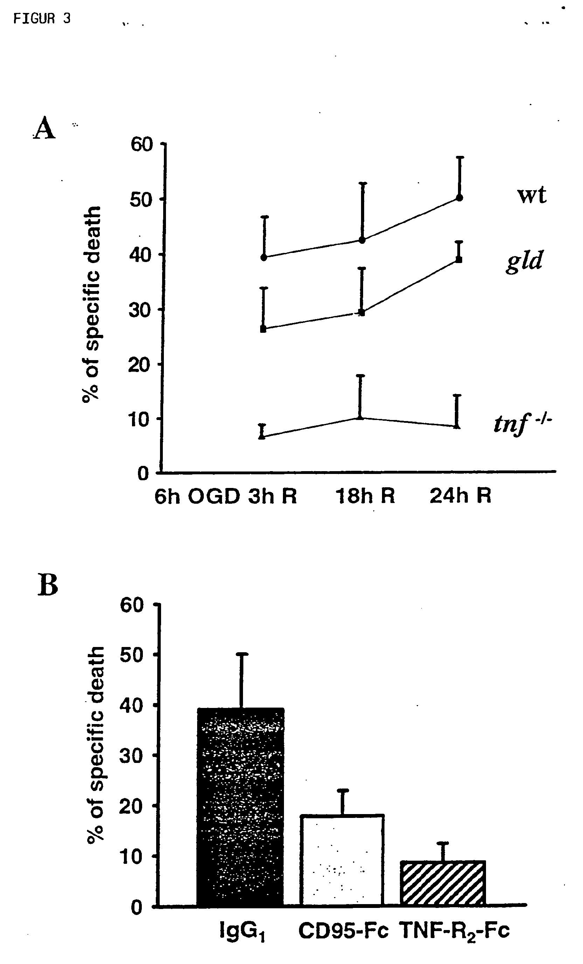

Es konnte in der vorliegenden Erfindung gezeigt werden, daß die beiden Liganden CD95L und TNF in synergistischer Weise den Zelltod im Anschluß an Ischämie auslösen. Diese den Zelltod fördernde Rolle von CD95L und TNF beruht nicht auf Veränderungen im Blutglucosegehalt oder hämodynamischen Parametern, die einen unspezifischen neuronalen Schutz zur Verfügung stellen könnten. Aus den Experimenten ergibt sich, daß CD95L und TNF sowohl bei der ischämischen als auch der entzündlichen Schädigung des Gehirns eine Rolle spielen. Bezüglich TNF oder einem funktionellen CD95L defiziente Neuronen (tnf$^{-/-}$-Neurone bzw. gld-Neurone) zeigten einen partiellen Schutz gegenüber dem Tod durch Ischämie (Fig. 3a). Dieser Schutz konnte auch in WT-Neuronen nachgewiesen werden, in denen TNF und CD95L durch als "Köder" eingesetzte Rezeptorproteine dem System entzogen worden waren. Der Schutz war in vitro in tnf$^{-/-}$-Neuronen höher als in gld-Neuronen. In der in vivo-Situation zeigten allerdings die Infarktvolumina in tnf$^{-/-}$- und gld-Mäusen keinen signifikanten Unterschied. Allerdings wurden Granulozyten zu dem ischämischen Bereich in tnf$^{-/-}$-Mäusen zu einem geringeren Ausmaß im Vergleich zu den gld-Mäusen rekrutiert. Die ähnliche Potenz von CD95L hinsichtlich der Förderung der ischämischen Schädigung kann durch dessen zusätzliche Fähigkeit zur Aktivierung der Maschinerie der zytotoxischen Granulozyten erklärt werden - Granulozyten vermitteln die direkte Zytolyse von CD95L+-Zellen. Jedenfalls zeigten CD95L und TNF eine synergistische Wirkung bei der Schädigung des Hirns nach einem Schlaganfall. Schließlich zeigte sich, daß der durch gld-, tnf$^{-/-}$- und gld/tnf$^{-/-}$ gezeigte neuronale Schutz (anhand des Infarktvolumens sichtbar gemacht; Fig. 1b) zu der Sterblichkeitsrate dieser Mäuse innerhalb des Beobachtungszeitraums parallel verlief (Fig. 1a). Somit führte die Verringerung der Hirnschädigung zu einer erhöhten Überlebensrate der Tiere. Der auffallende bei gld/tnf$^{-/-}$-Mäusen beobachtete neuronale Schutz zeigt, daß TNF-$\alpha$/TNF-Rezeptor und CD95L/CD95 pharmakologische Ziele bei der Prävention/Behandlung des Schlaganfalls darstellen. Heutzutage zielen Strategien hinsichtlich eines neuronalen Schutzes auf die Aufrechterhaltung der Lebensfähigkeit der ischämischen Neuronen solange bis eine Reperfusion normalerweise wieder etabliert werden kann. Auf eine zerebrale Reperfusion folgt jedoch eine Zerstörung und eine weitere Ausdehnung der Infarktzone. Somit kann davon ausgegangen werden, daß ein Schutz sowohl gegenüber Schäden durch Reperfusion als auch ischämischen Zelltod als eine ideale Therapie des Schlaganfalls angesehen werden kann. Aufgrund der in der vorliegenden Erfindung erhaltenen Ergebnisse kann davon ausgegangen werden, daß dies dadurch erreicht werden kann, daß die biologische Wirkung von TNF-$\alpha$ und CD95L gehemmt bzw. neutralisiert wird.

Somit betrifft die vorliegende Erfindung ein Arzneimittel, das eine oder mehrere Verbindungen gemäß den Patentansprüchen enthält, welche die biologischen Wirkungen von TNF-$\alpha$ und CD95L hemmen.

Der hier verwendete Ausdruck "Verbindungen, welche die biologischen Wirkungen von TNF-$\alpha$ und CD95L hemmen" betrifft alle Verbindungen, welche die biologischen Wirkungen von TNF-$\alpha$ und CD95L vollständig oder zumindest wesentlich hemmen bzw. neutralisieren können. Die Wirkung kann beispielsweise darauf beruhen, daß die Bindung von TNF-$\alpha$ und CD95L an ihre natürlichen Rezeptoren und somit die dadurch bewirkten Signalübertragungen unterdrückt werden. Dies kann beispielsweise dadurch erreicht werden, daß Antikörper verwendet werden, die derart entweder an TNF-$\alpha$ selbst oder den TNF-$\alpha$-Rezeptor binden bzw. an CD95L selbst oder CD95, daß die Bindung von TNF-$\alpha$ und CD95L an die Rezeptoren blockiert wird. Auch können kleinmolekulare Substanzen verwendet werden, die mit der Bindung von TNF-$\alpha$ an den TNA-$\alpha$-Rezeptor bzw. mit der Bindung von CD95L an den CD95L-Rezeptor interferieren. Des weiteren können auch Analogstoffe, beispielsweise des extrazellulären, löslichen Teils des Rezeptors oder ein verändertes TNF-$\alpha$ bzw. CD95L, beispielsweise ein kompetitiver oder nichtkompetitiver Antagonist, verwendet werden, wobei durch die (bevorzugte) Bindung von TNF-$\alpha$ bzw. CD95L an das Rezeptoranalogon bzw. die (bevorzugte) Bindung des Antagonisten an den natürlichen Rezeptor die Bindung des biologisch aktiven TNF-$\alpha$ bzw. CD95L an den natürlichen Rezeptor verringert oder ganz aufgehoben wird.

In einer bevorzugten Ausführungsform des erfindungsgemäßen Arzneimittels sind die Verbindungen, welche die Bindung von TNF-$\alpha$ und CD95L an ihre natürlichen Rezeptoren verhindern, ein neutralisierender anti-TNF-$\alpha$-Antikörper und ein neutralisierender anti-CD95L-Antikörper oder Fragmente davon, vorzugsweise monoclonale Antikörper oder Fragmente davon.

Verfahren zur Gewinnung solcher Antikörper sind dem Fachmann bekannt und umfassen beispielsweise bezüglich polyclonaler Antikörper die Verwendung von TNF-$\alpha$ bzw. CD95L oder eines Fragments davon oder eines von der Aminosäuresequenz abgeleiteten synthetischen Peptids als Immunogen zur Immunisierung geeigneter Tiere und die Gewinnung von Serum. Verfahren zur Herstellung monoclonaler Antikörper sind dem Fachmann ebenfalls bekannt. Dazu werden beispielsweise Zell-Hybride aus Antikörper produzierenden Zellen und Knochenmark-Tumorzellen hergestellt und cloniert. Anschließend wird ein Clon selektiert, der einen Antikörper produziert, der für TNF-$\alpha$ bzw. CD95L spezifisch ist. Dieser Antikörper wird dann hergestellt. Beispiele von Zellen, die Antikörper produzieren, sind Milzzellen, Lymphknotenzellen, B-Lymphozyten etc.. Beispiele von Tieren, die zu diesem Zweck immunisiert werden können, sind Mäuse, Ratten, Pferde, Ziegen und Kaninchen. Die Myelomzellen lassen sich aus Mäusen, Ratten, Menschen oder anderen Quellen erhalten. Die Zellfusion kann man beispielsweise durch das allgemein bekannte Verfahren von Köhler und Milstein durchführen. Die durch Zellfusion erhaltenen Hybridome werden mittels TNF-$\alpha$ bzw. CD95L nach dem Enzym-Antikörper-Verfahren oder nach einem ähnlichen Verfahren abgesucht. Clone werden beispielsweise mit dem Grenz-Verdünnungsverfahren erhalten. Die erhaltenen Clone werden BALB/c-Mäusen intraperitoneal implantiert, nach 10 bis 14 Tagen wird der Ascites der Maus entnommen, und der monoclonale Antikörper durch bekannte Verfahren (beispielsweise Ammoniumsulfatfraktionierung, PEG-Fraktionierung, Ionenaustauschchromatographie, Gelchromatographie oder Affinitätschromatographie) gereinigt. In dem erfindungsgemäßen Arzneimittel kann der gewonnene Antikörper direkt verwendet werden oder es kann ein Fragment davon verwendet werden. In diesem Zusammenhang bedeutet der Begriff "Fragment" alle Teile des monoclonalen Antikörpers (z.B. Fab-, Fv- oder "single chain Fv"-Fragmente), welche die gleiche Epitopspezifität wie der vollständige Antikörper aufweisen.

In einer besonders bevorzugten Ausführungsform sind die in dem erfindungsgemäßen Arzneimittel enthaltene monoclonale Antikörper aus einem Tier (z.B. Maus) stammende Antikörper, humanisierte Antikörper oder chimäre Antikörper oder Fragmente davon. Chimäre, menschlichen Antikörper ähnelnde oder humanisierte Antikörper besitzen eine herabgesetzte potentielle Antigenität, jedoch ist ihre Affinität gegenüber dem Ziel nicht herabgesetzt. Die Herstellung von chimären und humanisierten Antikörpern bzw. von den menschlichen Antikörpern ähnelnden Antikörpern wurde ausführlich beschrieben (siehe beispielsweise Queen et al., Proc. Natl. Acad. Sci. USA 86 (1989), 10029; und Verhoeyan et al., Science 239 (1988), 1534). Humanisierte Immunglobuline weisen variable Grundgerüstbereiche auf, die im wesentlichen von einem humanen Immunglobulin stammen (mit der Bezeichnung Akzeptor-Immunglobulin) und die Komplementarität der determinierenden Bereiche, die im wesentlichen von einem nicht-menschlichen Immunglobulin (z.B. von der Maus) stammen (mit der Bezeichnung Donor-Immunglobulin). Die (der) konstante(n) Bereich(e) stammt/stammen, falls vorhanden, auch im wesentlichen von einem menschlichen Immunglobulin. Bei der Verabreichung an menschliche Patienten bieten die erfindungsgemäßen humanisierten (sowie die menschlichen) anti-TNF-$\alpha$-Antikörper bzw anti-CD95L-Antikörper eine Reihe von Vorteilen gegenüber Antikörpern von Mäusen oder anderen Spezies: (a) das menschliche Immunsystem sollte das Grundgerüst oder den konstanten Bereich des humanisierten Antikörpers nicht als fremd erkennen und daher sollte die Antikörper-Antwort gegen einen solchen injizierten Antikörper geringer ausfallen als gegen einen vollständig fremden Maus-Antikörper oder einen partiell fremden chimären Antikörper; (b) da der Effektorbereich des humanisierten Antikörpers menschlich ist, dürfte er mit anderen Teilen des menschlichen Immunsystems besser interagieren, und (c) injizierte humanisierte Antikörper weisen eine Halbwertszeit auf, die im wesentlichen zu der von natürlich vorkommenden menschlichen Antikörpern äquivalent ist, was es erlaubt, kleinere und weniger häufige Dosen im Vergleich zu Antikörpern anderer Spezies zu verabreichen.

In einer weiteren bevorzugten Ausführungsform enthält das erfindungsgemäße Arzneimittel Verbindungen, die ein löslicher TNF-$\alpha$-Rezeptor und ein löslicher CD95-Rezeptor oder Teile davon sind. Der Fachmann weiß, wie er solche Verbindungen bereitstellen kann. Insbesondere wird auf das deutsche Patent P 44 12 177 des Anmelders und die Arbeit von Dhein et al., Nature 373 (1995), 438-441 verwiesen.

Die Verbindungen, welche die biologische Wirkung von TNF-$\alpha$ und CD95L neutralisieren, werden gegebenenfalls in Kombination mit einem pharmazeutisch verträglichen Träger verabreicht. Beispiele für geeignete Träger sind dem Fachmann bekannt und dazu gehören beispielsweise Phosphat-gepufferte Salzlösungen, Wasser, Emulsionen, Netzmittel etc.. Die Verabreichung der diese Verbindungen enthaltenden Arzneimittel kann oral oder, vorzugsweise, parenteral erfolgen. Zu den Verfahren für die parenterale Verabreichung gehören die topische, intra-arterielle, intramuskuläre, subkutane, intramedulläre, intrathekale, intraventrikuläre, intravenöse, intraperitoneale oder intranasale Verabreichung. Die geeignete Dosierung wird von dem behandelnden Arzt bestimmt und hängt von verschiedenen Faktoren ab, beispielsweise von dem Alter, dem Geschlecht, dem Gewicht des Patienten, dem Stadium und Ausmaß des Schlaganfalles oder Herzinfarkts, der Art der Verabreichung etc..

Eine weitere Ausführungsform der vorliegenden Erfindung betrifft die Verwendung der vorstehenden Verbindung(en), die biologische Wirkung von TNF-$\alpha$ und die biologische Wirkung von CD95L zu hemmen bzw. zu neutralisieren zur Prävention oder Behandlung eines Schlaganfalls oder Herzinfarkts. Bezüglich der für diese Verwendung geeigneten Verbindungen sind alle vorstehend beschriebenen Verbindungen geeignet, vorzugsweise ein neutralisierender anti-TNF-$\alpha$-Antikörper und ein neutralisierender anti-CD95L-Antikörper oder Fragmente davon. Besonders bevorzugt sind monoclonale Antikörper oder Fragmente davon, und am meisten bevorzugt sind aus einem Tier stammende Antikörper, humanisierte Antikörper oder chimäre Antikörper oder Fragmente davon. In einer alternativen Ausführungsform sind die Verbindungen ein löslicher TNF-$\alpha$-Rezeptor und ein löslicher CD95-Rezeptor oder Teile davon.

Für die Verwendung bei der Prävention oder Behandlung eines Herzinfarkts kann es - vor allem in Abhängigkeit von der Schwere des Krankheitsbilds - sinnvoll sein, die erfindungsgemäßen Verbindungen noch mit thrombolytischen Mitteln zu kombinieren. Geeignete thrombolytische Mittel sind auf dem Fachgebiet bekannt und umfassen beispielsweise Acetylsalicylsläure, Nitroglycerin, Cumarin, Heparin, Heparinoide, Fibrinolytika etc.

Ergänzend wird betont, daß sich ein erfindungsgemäßes Arzneimittel nicht nur dazu eignet, einer Ischämie im Gehirn bzw. einem Schlaganfall oder Herzinfarkt vorzubeugen, und / oder die dabei entstehenden Schädigungen zu verhindern oder zu reduzieren, sondern auch generell bei neurodegenerativen Erkrankungen, wie Alzheimer, Parkinson und Rückenmark-Trauma, verwendet zu werden.

Die Figuren zeigen:

Figur 1: Ischaemische Hirnschädigungen sind in gld-, tnf-/- und gld/tnf-/--Mäusen verringert

(A) Sterblichkeit (in %) innerhalb von 24 Stunden nach Verschluß der mittleren zerebralen Arterie (MCA) in Wildtyp-(n=14), gld- (n=17), tnf$^{-/-}$- (n=13) und gld/tnf$^{-/-}$-(n=15) Mäusen.

(B) Infarktvolumen nach transienter fokaler Ischaemie in Wildtyp- (n=9), gld- (n=8), tnf$^{-/-}$ - (n=7) und gld/tnf$^{-/-}$- (n=8) Mäusen. Die Tiere wurden 90 min einem MCA-Verschluß unterworfen und wie beschrieben 24 Stunden reperfundiert. 20 $\mu$m dicke, jeweils 400 $\mu$m voneinander entfernte koronale Kryostat-Schnitte wurden Silber-gefärbt. Das Infarktvolumen wurde durch die numerische Integration von Bereichen mit deutlicher Blässe gegenüber der Dicke der Schnitte bestimmt. Die Daten sind als Mittelwerte $\pm$ S.E.M dargestellt. Die Signifikanz wurde durch Vergleich von gld-, tnf$^{-/-}$- und gld/tnf$^{-/-}$-Mäusen mit Wildtypmäusen unter Verwendung des "Mann Whitney's"-Tests bestimmt (P<0,01, P<001 bzw. P<0,001). (C) Die Bildanalyse der regionalen Infarkthäufigkeiten des kranzförmigen Bereichs bei Bregma -2,3 mm von Wildtyp-, gld-, tnf$^{-/-}$ und gld/tnf$^{-/-}$-Mäusen verdeutlicht eine relative Aussparung des motorischen und somatosensorischen Cortex und des Striatums in gld-Mäusen und des gesamten angrenzenden Neocortex, Striatums und Thalamus in tnf $^{-/-}$-Mäusen. In gld/tnf$^{-/-}$-Mäusen war der Hippocampus praktisch der einzige betroffene Bereich. MCx: motorischer Cortex; SSCx: somatosensorischer Cortex; Hc: Hippocampus; Th: Thalamus; St: Striatum.

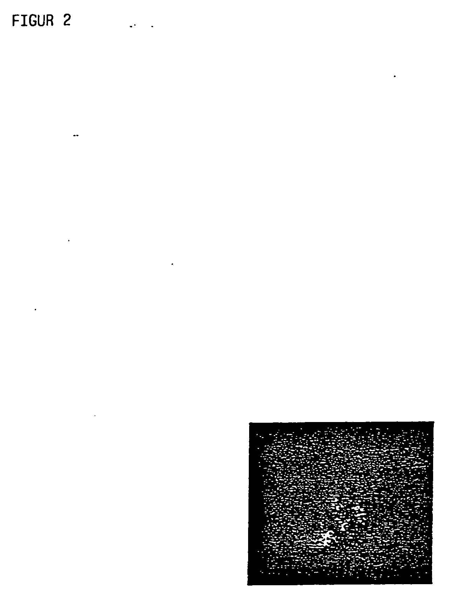

Figur 2: CD95L und TNF sind in der ischämischen Penumbra in der gleichen Zelle lokalisiert
Doppel-Immunfluoreszenzanalyse wurde an Gehirnschnitten von Wildtyp-Mäusen durchgeführt, die einem 90minütigen MCA-Verschluß und einer 24stündigen Reperfusion unterworfen worden waren. Zellen in der ischämischen Premubra waren hinsichtlich CD95L und TNF positiv (FITC: grün; Cy3: rot; beide zusammen: gelb)

Figur 3: Schutz gegenüber Tod durch Ischämie im in vitro-Modell für einen vaskulären Schlaganfall.

(A) Rinden-Neuronen von Wildtyp-, gld-, tnf$^{-/-}$ und gld/tnf$^{-/-}$-Mäusen wurden einem sechsstündigen Sauerstoff/ Glucose-Entzug (OGD) und ansteigenden Reperfusions-Perioden (R) unterworfen. Der spezifische Tod wurde nach 3, 18 und 24 Stunden Reperfusion (R) bestimmt. (B) Rinden-Neuronen von Wildtyptieren wurden vor der Induktion von 6 Stunden OGD und 18 Stunden Reperfusion mit CD95-Fc-Proteinen und TNF-R2-Fc-Proteinen (je 20 $\mu$g) inkubiert; der spezifische Tod wurde am Ende der Reperfusionsperiode ermittelt. Die Inkubation mit Kontroll-Immunglobulin (IgG$_1$) hatte keine Auswirkung hinsichtlich der Neurotoxizität der Kulturen. Der Zelltod wurde mittels des "Trypanblau-Ausschluss"-Verfahrens abgeschätzt und ist jeweils als Mittelwert $\pm$ Standard-abweichung (n=3) angegeben. Der spezifische Zelltod wurde wie in den nachstehenden Beispielen beschrieben berechnet.

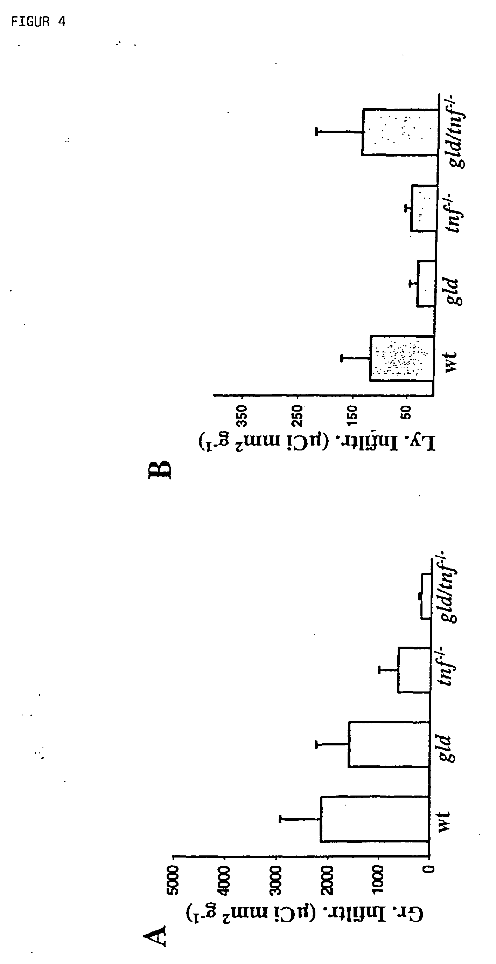

Figur 4: Infiltration des ischämischen Gehirns mit an Entzündunasnrozessen beteiligten Zellen
Infiltration mit Granulozyten (Gr.Infiltr.; A) oder Lymphozyten (Ly.Infiltr.; B) des ischämischen Gehirns von Wildtyp-, gld-, tnf$^{-/-}$ und gld/tnf$^{-/-}$-Mäusen (n jeweils = 3) wurde mittels Autoimmunradiographie quantifiziert. Gehirnschnitte vom Gehirn, das einer herdförmigen Ischämie unterworfen worden war ( 90 min MCA-Verschluß und 25 Stunden Reperfusion) wurden mit primären Antiseren gegen GR1 (für Granulozyten) und CD3 (für Lymphozyten) inkubiert. Die Anfärbung wurde mittels Autoradiographie bestimmt. Entzündliche Infiltrate in der ischämischen Hemisphäre wurden durch Messung des Bereichs und der optischen Dichte (OD) der Infiltrate quantifiziert. Die Ergebnisse sind als Mittelwerte $\pm$ S.E.M (n=3) dargestellt.

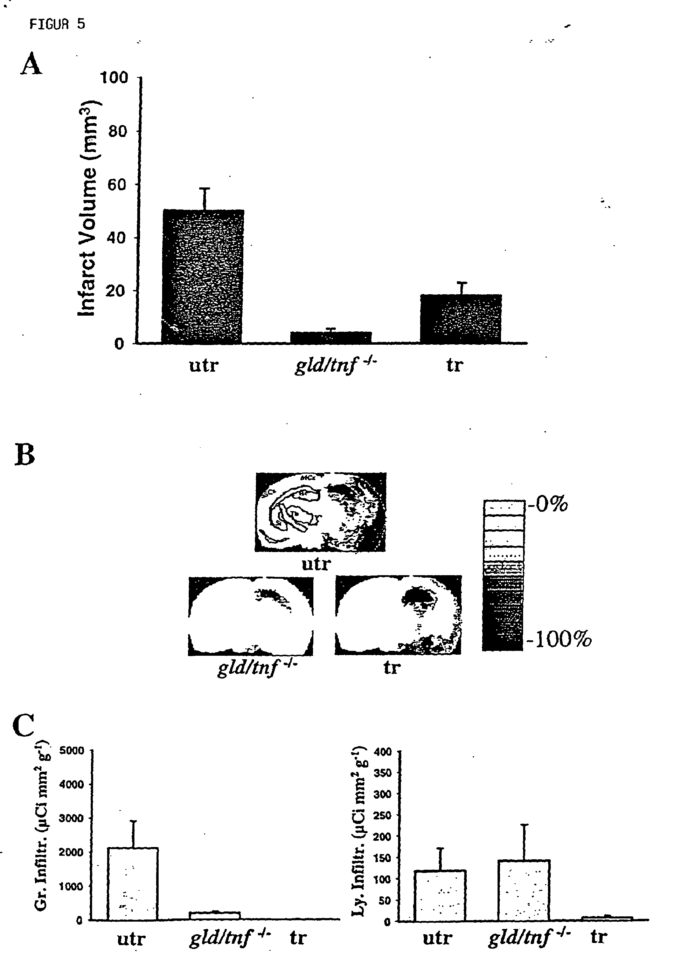

Figur 5: Infarktausdehnung und entzündliche Infiltrate des ischämischen Gehirns werden durch anti-TNF- und anti-CD95L-Antikörper signifikant verringert
Unbehandelte (n=9; utr) oder mit Antikörpern gegen TNF und CD95L (n=8; tr) behandelte Tiere und gld/tnf$^{-/-}$-Tiere (n=8) wurden einem 90minütigen MCA-Verschluß und einer 24stündigen Reperfusion unterworfen. Kranzförmige Kryostat-Schnitte (Dikke: 20 $\mu$m; Abstand zueinander jeweils 400 $\mu$m) wurden Silber-gefärbt. Das Infarktvolumen wurde durch numerische Integration der Bereiche mit deutlicher Blässe mit der Schnittdicke bestimmt. Die Daten sind als Mittelwerte $\pm$ S.E.M dargestellt. Die Signifikanz wurde durch Vergleich der "tr"- und gld/tnf$^{-/-}$-Mäuse mit "utr"-Mäusen unter Verwendung des "Mann Whitney's"-Tests (P<0,01 bzw. P<0,0001) ermittelt. (B) Bildanalyse der regionalen Infarkthäufigkeiten des kranzförmigen Schnitts bei Bregma -2,3 mm von "utr"-, "tr"- und gld/tnf$^{-/-}$-Mäusen ergab eine relative Aussparung des motorischen und somatosensorischen Cortex und des Striatums in "tr"-Mäusen. Im gld/tnf$^{-/-}$-Mäusen war der Hippocampus praktisch der einzige betroffene Bereich. MCx: motorischer Cortex; SSCx, somatosensorischer Cortex; Hc: Hippocampus; Th: Thalamus; St: Striatum; C: Anzahl der Granulozyten (Gr.Infiltr.) und Lymphozyten (Ly.Infiltr.) in der ischämischen Hemisphäre wurde mittels Autoimmunradiographie bestimmt. Gehirnschnitte von Tieren, die einer herdförmigen Ischämie unterworfen worden waren (90 min MCA-Verschluß und 24 Stunden Reperfusion) wurden mit primären Antiseren gegen GR1 (für Granulozyten) und CD3 (für Lymphozyten) inkubiert. Die Anfärbungen wurden mittels Autoradiographie sichtbar gemacht. Entzündliche Infiltrate in der ischämischen Hemisphäre wurden durch Messung des Bereichs und der optischen Dichte (OD) der Infiltrate quantifiziert. Die Daten sind als Mittelwerte $\pm$ S.E.M (n=3) dargestellt.

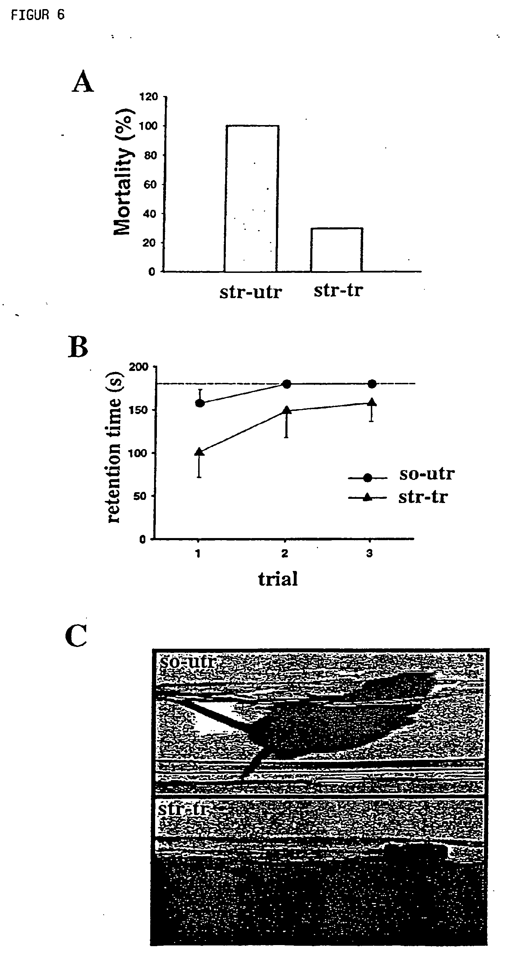

Figur 6: Neutralisation von CD95L und TNF verringert die Sterblichkeit und verbessert die motorischen Leistungen der Tiere nach Schlaganfall

(A) Sterblichkeit (in %) innerhalb von drei Tagen nach MCA-Verschluß bei unbehandelten Tieren (n=5; str-utr) oder mit anti-TNF- und anti-CD95L-Antikörpern behandelten Tieren (n=10; str-utr). (B) Haltezeiten auf einem Stab mit einer allmählich ansteigenden Rotationsgeschwindigkeit für unbehandelte scheinoperierte (so-utr; n=6) und behandelte Schlaganfall-Tiere (str-tr; n=5). (C) Erste Erfahrungen der "so-utr"- und "str-tr"-Mäuse hinsichtlich Schwimmen.

Die nachstehenden Beispiele erläutern die Erfindung.

**Beispiel 1: Allgemeine Verfahren**

(A) Ischämisches Tiermodell: In Wildtyp- (n=14), gld- (n=17), tnf$^{-/-}$ (n=13) und gld/tnf$^{-/-}$ (n=15)Mäusen, die alle hinsichtlich des Alters (Mittelwert: 100 Tage) und des Gewichts (Mittelwert: 24 g) abgestimmt waren, wurde eine herdförmige cerebrale Ischämie durch MCA-Verschluß wie von Zea Longa et al., Stroke 20 (1989), 84 beschrieben induziert. Ein chirurgischer Nylonfaden wurde vom Lumen der gemeinsamen Halsschlagader bis zur vorderen Cerebralarterie zur Blockierung des Ursprungs für die MCA 90 Minuten geschlungen. Durch Entfernung des Nylonfadens wurde der Blutfluß wieder hergestellt. Mittels Ketamin/ROMPOM (je 150 mg/kg Körpergewicht) wurde eine tiefe Narkose durchgeführt. Die Tiere wurden unter Narkose gehalten und die rektale Temperatur wurde bei bzw. im Bereich von 37°C mittels Hitze-erzeugenden Lampen sowohl während der chirurgischen Eingriffe als auch des Zeitraums des MCA-Verschlusses gehalten. Nach verschiedenen Reperfusionsperioden wurden die Tiere erneut tief narkotisiert und durch Abtrennen des Kopfes getötet. Zum Erhalt physiologischer Parameter wurde in die rechte Oberschenkelarterie unter andauernder Anästhesie eine Kanüle eingeführt, der Blutdruck kontinuierlich aufgezeichnet und Proben bezüglich des Blut-Gasgehalts und Glucose 15 min vor, 1 Stunde nach Beginn und 30 min nach Beendigung des MCA-Abschlusses entnommen. Das Geschlecht der Mäuse variierte je nach Verfügbarkeit. Der Ausgang des Infarkts wurde durch das Geschlecht der Tiere jedenfalls nicht beeinflußt. Alle Mäuse hatten einen C57BL/6-Hintergrund, um so die bekannten Unterschiede bezüglich der Empfänglichkeit für einen Infarkt in Abhängigkeit vom jeweiligen Mäusestamm zu vermeiden (Connolly et al., Neurosurgery 38 (1996), 523). Für die Behandlungsexperimente wurden jeweils 50 $\mu$g anti-CD95L-Antikörper MFL3 und anti-TNF-Antkörper Vlq entweder i.v. oder i.p. 15 min und 24 Stunden nach MCA-Verschluß injiziert.

(B) Messung der Infarktausdehnung: Die Mäuse wurden einem MCA-Filamentverschluß für 90 min unterzogen und es erfolgte wie beschrieben eine Reperfusion für 24 Stunden. Die Vorderhirne wurden geschnitten und kranzförmige Kryostat-Schnitte (Dicke: 20 $\mu$m, jeweils 400 $\mu$m voneinander entfernt) wurden Silber-gefärbt. Kurz zusammengefaßt wurde so vorgegangen: Schnitte wurden mit einer Silbernitrat/Lithiumcarbonat-Lösung 2 min imprägniert und mit einer Hydrochinon/Formaldehyd-Lösung 3 min entwickelt. Gefärbte Schnitte wurden direkt gescannt (MCID-M4, 3.0. Das Infarktyolumen wurde durch numerische Integration des gescannten Bereichs mit ausgeprägter Blässe bestimmt (korrigiert für Gehirnödem x Schnittdicke unter Verwendung digitaler Planimetrie (Swanson et al., J.Cereb.Blood Flow Metab. 10 (1990), 290)). Alle Daten sind als Mittelwerte $\pm$ S.E.M (mittlere Standardabweichung) angegeben. Die Signifikanz wurde mittels des MANN-WITHNEY's t-Tests ermittelt. Zur Herstellung von Infarkthäufigkeitskarten wurden die entsprechenden Schnitte gescannt, Infarkte dargestellt und auf eine Maske projiziert. Die Durchschnittsermittlung erfolgte mit einem "Scion Image ß 3.b".

(C) Immunhistochemische Analyse der CD95L- und TNF-Expression Kranzförmige Kryostat-Schnitte (20 $\mu$m) von Wildtypmäusen, bei denen ein 90minütiger MCA-Verschluß und eine 24stündige Reperfusion durchgeführt worden waren, wurden für eine immunhistochemische Analyse vorbereitet. Die Schnitte wurden mit einem monoclonalen Antikörper gegen CD95L (P62) oder einem monoclonalen Antikörper gegen TNF (Hartung) inkubiert. Immunreaktionen mit CD95L und TNF wurden entweder mit einem sekundären polyclonalen FITC-markierten oder einem sekundären monoclonalen Cy3-markierten Antikörper sichtbar gemacht.

(D) Nachweis der Infiltration von an Entzündunctsprozessen beteiligten Zellen: Kranzförmige Kryostat-Schnitte (20 $\mu$m) von Wildtyp-, gld-, tnf$^{-/-}$- und gld/tnf$^{-/-}$-Mäusen, bei denen ein 90minütiger MCA-Verschluß und eine 24stündige Reperfusion durchgeführt worden waren, wurden für eine Immunautoradiographie vorbereitet. Die Schnitte wurden

EP 1 235 592 B1

24 Stunden mit dem monoclonalen Antikörper gegen GR1 (Ly-1) oder einem monoclonalen Antikörper gegen CD3 (Chemicon) inkubiert. Danach wurden die Schnitte mit einem [125I]-markierten sekundären Antikörper inkubiert. Das Radioautogramm wurde (zusammen mit einem [125I]-Standard-Set) auf einem Kodak "MinR1"-Röntgenfilm erstellt (Exposition: 21 Tage). Granulozyten- oder Lymphozyten-Infiltration in der ischämischen Hemisphäre wurden durch Messung der optischen Dichte und des Bereichs der Infiltrate mit einem Bildanalyse-System bestimmt (MCID).

(E) Zellkultur und experimentelle in vitro-Behandlung: Primäre neuronale Kulturen wurden von fötalen Mäusen (vom 15. bis 17. Tag) präpariert. Kurz zusammengefaßt wurde folgendermaßen vorgegangen: Rindenneuronen wurden nach Zerreiben im MEM-Medium mit 20% Pferdeserum, 25 mM Glucose und 2 mM L-Glutamin erhalten. Danach folgte eine 30minütige Spaltung in 0,025% Trypsin/Kochsalzlösung. Die Zellen wurden in Platten mit 24 Vertiefungen, die mit Polyornithin beschichtet waren, ausplattiert. Nach vier Tagen wurden die Zellen für weitere vier Tage zur Hemmung der Proliferation nicht-neuronaler Zellen mit Citosin-Arabinosid (5 $\mu$M) behandelt. Danach wurden die Zellkulturen in MEM, 10% Pferdeserum, 5% fötalem Rinderserum, 25 mM Glucose und 2 mM L-Glutamin bei 37˚C in einem angefeuchteten $CO_2$-Inkubator (8%) gehalten. Die Neuronen wurden mindestens 8 Tage vor der experimentellen Verwendung in Kultur reifen gelassen. Der Anteil von Gliazellen in den Kulturen lag unter 10% (abgeschätzt mittels eines Antikörpers gegen "glial-fibrilary-acidic protein"(GFAP).

(F) Sauerstoff/Glucose-Entzug in vitro: Ein kombinierter Sauerstoff/Glucose-Entzug wurde durchgeführt. Das Kulturmedium wurde gegen MEM, 1% Pferdeserum und 2 mM L-Glutamin ausgetauscht. Die Kulturen wurden 6 Stunden bei 37˚C und 100% Feuchtigkeit in einer anaeroben Kammer gehalten, die das Gasgemisch $5\%H_2/85\%N_2/5\%CO_2$ enthielt. Der kombinierte Sauerstoff/Glucose-Entzug wurde durch Entfernung der Kulturen aus der Kammer und Zugabe von Pferdeserum, fötalem Rinderserum und Glucose bis zu einer Endkonzentration von 10%, 5% bzw. 25 mM beendet. Die Kulturen wurden dann bei 37˚C für weitere 3, 18 oder 24 Stunden in einem befeuchteten Inkubator inkubiert, der 8% $CO_2$ und atmosphärischen Sauerstoff enthielt. Humanes $IgG_1$, CD95-Fc und TNF-R2-Fc (jeweils 20$\mu$M) wurden 5 min vor der Induktion von OGD zu dem Kulturmedium gegeben. Der Prozentsatz an Zelltod wurde mittels des "Trypanblau-Ausschluß"-Verfahrens bestimmt und als % spezifischer Zelltod angegeben. Dieser wurde folgendermaßen berechnet:

$$\% \text{ spezifischer Zelltod} = \frac{(\text{bestimmter} - \text{spontaner Zelltod}}{(100 - \text{spontaner Zelltod})} \times 100$$

Der spontane Zelltod betrug 12% $\pm$ 0,9 für Neuronen der tnf-$\alpha$ "knockout"-Mäuse, 10% $\pm$ 0,73 für Neuronen von gld-Mäusen und 15% $\pm$ 0,87 für Neuronen von C57BL/6 Wildtypmäusen. Alle Daten sind als Mittelwerte $\pm$ Standardabweichung (n=3) angegeben.

(F) Motorische Koordination: 12 bis 16 Wochen alte männliche C57BL/6 Mäuse wurden auf einen fixierten horizontalen Holzstab oder Plexiglasstab gesetzt und die Zeit, in der das Tier auf dem Stab blieb, wurde gemessen. Für die Experimente mit dem sich drehenden Stab wurde die Maus auf eine mit feinem Splitt beschichtete Plastikrolle gesetzt, die innerhalb von 5 min. von 4 auf 40 UpM beschleunigt wurde. Die Haltezeiten wurden innerhalb eines Zeitraums bis zu 180 sec aufgezeichnet.

**Tabelle 1**

**Physiologische Variable vor, während und nach MCA-Verschluß in Wildtyp (C57BL/6)-, gld-, tnf$^{-/-}$- und gld/tnf$^{-/-}$-Mäusen.**

|  | Tier | vor | während | nach |
|---|---|---|---|---|
| MABP (mmHg) | Wildtyp | 95$\pm$8 | 93$\pm$10 | 95$\pm$12 |
|  | gld | 92$\pm$12 | 95$\pm$8 | 95$\pm$8 |
|  | tnf$^{-/-}$ | 77,5$\pm$6 | 72$\pm$9 | 105$\pm$5 |
|  | gld/tnf$^{-/-}$ | 75$\pm$4 | 82$\pm$13 | 84$\pm$12 |
| Arterieller pH | Wildtyp | 7,22$\pm$0,05 | 7,17$\pm$0,04 | 7,2$\pm$0,02 |
|  | gld | 7,3$\pm$0,07 | 7,22$\pm$0,09 | 7,23$\pm$0,02 |
|  | tnf-/- | 7,25$\pm$0,04 | 7,25$\pm$0,06 | 7,21$\pm$0,01 |

8

(fortgesetzt)

**Physiologische Variable vor, während und nach MCA-Verschluß in Wildtyp (C57BL/6)-, gld-, tnf$^{-/-}$- und gld/tnf$^{-/-}$-Mäusen.**

| Tier | vor | während | nach |
|---|---|---|---|
| gld/tnf$^{-/-}$ | 7,16±0,07 | 7,14±0,11 | 7,13±0,08 |
| **Arterieller PaCO$_2$ (Torr)** | | | |
| Wildtyp | 53,9±5 | 58,4±4 | 44,5±3 |
| gld | 48,7±5 | 51,4±11 | 49,9±1 |
| tnf$^{-/-}$ | 54,4±1 | 47,2±1 | 53,6±1 |
| gld/tnf$^{-/-}$ | 64,3±7 | 59,5±7 | 57,8±9 |
| **Arterieller PaO$_2$ (Torr)** | | | |
| Wildtyp | 152±26 | 82±6 | 124±11 |
| gld | 158±21 | 96±18 | 86±6 |
| tnf-/- | 118±14 | 126±14 | 98±6 |
| gld/tnf$^{-/-}$ | 103±8 | 84±10 | 115±6 |
| **Hämatocrit** | | | |
| Wildtyp | 35±5 | 45±1 | 39±4 |
| gld | 42±4 | 40±2 | 36±4 |
| tnf-/- | 41±1 | 46±2 | 42±1 |
| gld/tnf$^{-/-}$ | 40±6 | 39±8 | 35±8 |
| **Blutglucose (mg/dl)** | | | |
| Wildtyp | 206±17 | 225±17 | 217±27 |
| gld | 153±23 | 170±21 | 133±21 |
| tnf-/- | 182±16 | 242±44 | 231±25 |
| gld/tnf$^{-/-}$ | 216±50 | 171±43 | 187±55 |

MABP: mittlerer arterieller Blutdruck; Blutgase (pH, PaCO$_2$ und PaO$_2$) und Blutglucose wurden 15 min vor, 1 Stunde nach Beginn und 30 min nach Beendigung des MCA-Verschlusses gemessen. Die Daten sind als Mittelwerte ± Standardabweichung (alle Gruppen, n=4) angegeben. Die Signifikanz wurde durch Vergleich der gld-, tnf$^{-/-}$ und gld/tnf$^{-/-}$-Mäuse mit Wildtyp- Mäusen mittels des Student t- Tests (P<0,05) gemessen.

**Beispiel 2: CD95L und TNF fördern synergistisch den Zelltod nach Hirn-Ischämie**

Zur Untersuchung der Rolle von TNF und CD95L und ihrer möglichen Interaktion bei den Schäden durch Ischämie im Gehirn wurden Mäuse, die eine gezielte Disruption des tnf-Gens tnf$^{-/-}$ trugen, Mäuse mit einem mutierten CD95L mit der blockierten Fähigkeit zur erfolgreichen Interaktion mit CD95 (gld), Mäuse mit einem mutierten CD95L und defizient für TNF (gld/tnf$^{-/-}$) und Wildtypmäuse (WT), die alle einen C57BL/6 Hintergrund aufwiesen, verwendet. tnf$^{-/-}$ und gld-Mäuse zeigten keine Entwicklungsanomalien und nur spezifische Defekte hinsichtlich Immunantworten. gld/tnf$^{-/-}$-Mäuse zeigten keine strukturellen oder morphologische Anomalien und die mittels "Nissl"-Färbung der koronalen Kryostat-Schnitte bestimmte Gehirnanatomie erschien normal. WT-, gld-, tnf$^{-/-}$ und gld/tnf$^{-/-}$-Mäuse wurden einem 90minütigen MCA-Verschluß unterworfen und der vor, während und nach dem MCA-Verschluß gemessene Glucosegehalt schwankte nicht signifikant zwischen den einzelnen Tiergruppen (siehe Tabelle 1) . Das von Wildtyptieren gezeigte mittlere Infaktvolumen war in guter Übereinstimmung mit dem bei anderen Gruppen in ähnlichen Experimenten erhaltenen. Daten von Mäusen, die keine ischämische Läsion zeigten, oder von Mäusen, die vor Beendigung des Beobachtungszeitraums starben (41% Wildtyp-, 29% gld- und 17% tnf$^{-/-}$-Mäuse; Figur 1a), wurden nicht in die Analysen des Infarktvolumens miteinbezogen. In gld- und tnf$^{-/-}$-Mäusen war das Infarktvolumen im Vergleich zu Wildtypmäusen signifikant um etwa 54% bzw. 67% verringert (23,23±4,97 mm$^3$, n=8 und 16,44±7,24 mm$^3$, n=7 gegenüber 50,11±8,38 mm$^3$, n=9; für beide P<0, 01; Fig.1b). Überraschenderweise war der neuronale Schutz bei Fehlen von sowohl CD95L und TNF sehr stark verbessert. gld/tnf$^{-/-}$-Mäuse zeigten ein mittleres Infarktvolumen, das im Vergleich zu Wildtypmäusen signifikant geringer war (3,97±1,52 mm$^3$, n=8 bzw. 50,11±8,38 mm$^3$, n=9; P<0,0001; Fig.1b).

Die regionale Infarktverteilung in der coronalen Ebene wurde mittels der Durchschnittsermittlung der Infarktbereiche (coronaler Schnitt bei Bregma -2,3 mm) analysiert. Die erhaltene Häufigkeitsdichte-Karte zeigt eine relative Aussparung des motorischen und somatosensorischen Cortex und des Striatums in gld-Mäusen und eine relative Aussparung des gesamten angrenzenden Neocortex, des Striatums und des Thalamus in tnf$^{-/-}$-Mäusen (Fig. 1c). In gld/tnf$^{-/-}$-mäusen waren das Striatum, der Cortex und der Thalamus durch den ischämischen Insult nicht betroffen und der Schaden blieb hauptsächlich auf den Hippocampus beschränkt (Fig. 1c).

CD95L und TNF werden in Neuronen der ischämischen Penumbra exprimiert. Um zu untersuchen, ob sie auf derselben Zelle lokalisiert sind, wurde die Expression von CD95L und TNF in Hirnschnitten von Tieren, bei denen ein 90minütiger MCA-Verschluß und eine 24stündige Reperfusion durchgeführt worden waren, mittels Doppel-Immunfluoreszenz analysiert. In der ischämischen Penumbra war TNF auf CD95L exprimierenden Zellen lokalisiert (Fig. 2). Somit könnten TNF und CD95L ihre Expression gegenseitig erhöhen.

**Beispiel 3: CD95L und TNF fördern den neuronalen Tod und Entzündungen**

Um den der Förderung der ischämischen Hirnschädigung durch CD95L und TNF zugrunde liegenden Mechanismus näher untersuchen zu können, wurde das in vitro-Modell des Sauerstoff/Glucose-Entzugs (OGD) verwendet. OGD in primären neuronalen Kulturen stellt ein übliches in vitro-Modell für die Untersuchung von frühen Mechanismen bei Schädigungen durch einen vaskulären Schlaganfall in einem System dar, das ausschließlich aus Neuronen besteht. Von Wildtyp-, gld-, tnf$^{-/-}$ und gld/tnf$^{-/-}$-Mäusen erhaltene primäre corticale Neuronen wurden einem 6stündigen OGD und für 3, 18 bzw. 24 Stunden einer Reperfusion unterworfen. tnf$^{-/-}$-Neuronen waren im Vergleich zu Wildtyp-Neuronen gegenüber einer OGD/Reperfusion induzierten Schädigung im wesentlichen resistent; nur etwa 10% der tnf$^{-/-}$-Neuronen starben nach OGD/Reperfusion, während bis zu 47% der WT-Neuronen starben (Fig. 3a). Zu jedem untersuchten Zeitpunkt war das Ausmaß des spezifischen Zelltods der gld-Neuronen etwa 20% geringer im Vergleich zu WT-Neuronen (Fig. 3a). Von gld/tnf$^{-/-}$-Mäusen stammende neuronale Zellen konnten in Kultur nicht gezüchtet werden, was vermutlich durch die spezifische in vitro-Situation bedingt war.

tnf$^{-/-}$- und gld-Mäuse zeigten eine normale Entwicklung und Anatomie des Gehirns. Dieser offensichtlich normale "Cerebral-Phänotyp" könnte auf dem Vorhandensein eines kompensatorischen Mechanismus beruhen, der einen unspezifischen Schutz gegen Gehirn-Ischämie liefert. Um diese Möglichkeit ausschließen zu können, wurden 15 min vor der Induktion des OGD WT-Neuronen mit CD95-FC- und TNF-R2-Fc-Proteinen behandelt. Nach 6 Stunden OGD und 18 Stunden Reperfusion verringerte der Entzug entweder von CD95L oder TNF-$\alpha$ die Neurotoxizität der Kulturen um 25% bzw. 39% im Vergleich zu mit IgG$_1$ (Immunglobulin G$_1$) behandelten Kontrollen (Fig. 3b). Somit kann die Hemmung der Aktivität von TNF und CD95L den OGD/Reperfusion-induzierten neuronalen Tod spezifisch blockieren.

In der in vivo-Situation setzen kurz nach Verringerung des cerebralen Blutflusses Zytokinproduktion und molekulare adhäsive Ereignisse ein. Der von dem ischämischen Parenchym produzierte TNF ist an der endothelialen Zellexpression von zellulären adhäsiven Molekülen beteiligt, beispielsweise des interzellulären "adhesion molecule-1" (ICAM-1), des vasculären "cell adhesion molecule-1" (VCAM-1) und des "endothelial-leukocyte adhesion molecule-1" (E-selectin). Adhäsionsmoleküle erleichtern die Rekrutierung von an Entzündungsprozessen beteiligten Zellen zu der ischämischen Läsion. Für CD95L wurden in Tumoren ähnliche chemotaktische Eigenschaften beschrieben. Nach diesen Befunden stellte sich die Frage, ob zusätzlich zu der den Zelltod fördernden Rolle von CD95L und TNF im Anschluß an eine Ischämie diese beiden Moleküle auch durch Rekrutierung von an Entzündungsprozessen beteiligten Zellen an der ischämischen Schädigung beteiligt sind. Zur Beantwortung dieser Frage wurden in Gehirnschnitten von Wildtyp-, gld-, tnf$^{-/-}$- und gld/ tnf$^{-/-}$-Mäusen (n jeweils = 3), die einer fokalen Ischämie (90 min MCA-Verschluß und 24 Stunden Reperfusion) unterworfen worden waren, die Gegenwart von infiltrierenden Granulozyten und infiltrierenden Lymphozyten mittels Autoimmunradiographie analysiert. Kontrollfärbungen ohne den ersten Antikörper oder von Gehirnschnitten von scheinoperierten Tieren waren negativ. Die quantitative Analyse von Autoradiogrammen ergab ein abnehmendes Ausmaß der Granulozyten-Infiltration in gld-, tnf$^{-/-}$- und gld/tnf$^{-/-}$-Mäusen (vom höchsten bis zum niedrigsten Infiltrationsgrad angeordnet), die im Einklang mit dem Ausmaß der Gehirnschädigung stand (Fig. 4). Das Ausmaß der Lymphozyten-Infiltration war in WT- und gld/tnf$^{-/-}$-Mäusen vergleichbar und etwas erhöht im Vergleich zu gld- und tnf$^{-/-}$-Mäusen (Fig. 4). Diese Daten deuten auf eine synergistische chemotaktische Wirkung von TNF und CD95L in Richtung Granulozyten im Anschluß an Ischämie.

**Beispiel 4: Die Behandlung mit anti-TNF- und anti-CD95L-Antikörpern verringert die ischämischen Schädigungen**

Um die Erkenntnisse aus den genetisch veränderten Tieren in die Praxis umzusetzen, wurden im Anschluß an eine fokale Ischämie in vivo TNF und CD95L neutralisiert. In WT-Mäusen induzierte eine Injektion von anti-TNF- und anti-CD95L-Antikörpern i.v. (jeweils 50 $\mu$g) 15 min nach MCA-Verschluß eine etwa 60%ige Verringerung des Infarktvolumens im Vergleich zu unbehandelten Tieren (18,04$\pm$4,87 mm$^3$, n=8, in behandelten Mäusen versus 50,11$\pm$8,38 mm$^3$, n=9, P<0,01; Fig. 5a) Die regionale Infarktverteilung in der coronalen Ebene wurde durch die Ermittlung des Durchschnitts der Infarktzonen (coronaler Schnitt bei Bregma -2,3 mm) bestimmt. Die erhaltene Häufigkeitsdichte-Karte zeigt eine fast ausschließliche Beteiligung des Hippocampus und des piriformen Cortex in der ischämischen Läsion von behandelten Tieren ("stroke-tr"; Fig. 5b). In behandelten Tieren waren auch entzündliche Infiltrate kaum nachweisbar (Fig. 5c).

Die Funktionalität der geretteten Neuronen wurde durch Tests hinsichtlich der motorischen Koordination von behandelten Mäusen nach drei Tagen Reperfusion untersucht. Dazu wurden 15 min und 24 Stunden nach MCA-Verschluß anti-TNF- und anti-CD95L-Antikörper (jeweils 50 µg) i.p. injiziert. Alle unbehandelten, einer fokalen Ischämie unterworfenen Tiere starben vor Beendigung dieses Zeitraums ("stroke-utr"; n=5), während die Mortalität in der behandelten Gruppe ("stroke-tr"; n=10) nur 30% betrug (Fig. 6a). Sechs scheinoperierte unbehandelte Tiere ("so-utr") und fünf behandelte Mäuse, bei denen ein 90minütiger MCA-Verschluß und eine dreitägige Reperfusion durchgeführt worden waren, wurden auf einem rotierenden Stab und stationären Stäben getestet. Das von den ischämischen behandelten Tieren gezeigte Haltevermögen auf dem rotierenden Stab unterschied sich nicht signifikant von dem der scheinoperierten Tiere (Fig. 6b/c). Die motorische Koordination auf stationären Stäben verlief zu diesen Ergebnissen parallel. Die Aufrechterhaltung der die Achse betreffenden Balance war in "so-utr"- und "stroke-utr"-Tieren vergleichbar. Sogar Tiere mit einer geringen Haltezeit auf dem rotierenden Stab zeigten dann, wenn sie zum ersten Mal in Wasser gebracht wurden, eine unbeeinträchtigte die Achse betreffende Balance.

**Patentansprüche**

1. Arzneimittel, eine oder mehrere Verbindungen enthaltend, welche die biologische Wirkung von TNF$\alpha$ und CD95L hemmen, ausgewählt aus

   - einem neutralisierenden anti-TNF$\alpha$-Antikörper und einem neutralisierenden anti-CD95L-Antikörper oder Fragmenten davon oder
   - einem löslichen TNF$\alpha$-Rezeptor und einem löslichen CD95-Rezeptor oder Teilen davon.

2. Arzneimittel nach Anspruch 1, wobei der Antikörper ein monoclonaler Antikörper oder ein Fragment davon ist.

3. Arzneimittel nach Anspruch 2, wobei der monoclonale Antikörper ein aus einem Tier stammender Antikörper, ein humanisierter Antikörper oder ein chimärer Antikörper oder ein Fragment davon ist.

4. Arzneimittel nach einem der Ansprüche 1 bis 3, ferner ein thrombolytisches Mittel enthaltend.

5. Verwendung der in den Ansprüchen 1 bis 4 definierten Verbindungen zur Herstellung eines Arzneimittels zur Prävention oder Behandlung eines Schlaganfalles, Herzinfarktes oder einer degenerativen Erkrankung, insbesondere Alzheimer, Parkinson und Rückenmark-Trauma.

**Claims**

1. A medicament comprising one or more compounds which inhibit the biological effect of TNF-$\alpha$ and CD95L, selected from

   - a neutralizing anti-TNF-$\alpha$ antibody and a neutralizing anti-CD95L antibody or fragments thereof, or
   - a soluble TNF-$\alpha$ receptor and a soluble CD95 receptor or portions thereof.

2. The medicament according to claim 1, wherein the antibody is a monoclonal antibody or a fragment thereof.

3. The medicament according to claim 2, wherein the monoclonal antibody is an antibody derived from an animal, a humanized antibody or a chimeric antibody or a fragment thereof.

4. The medicament according to any one of claims 1 to 3, further comprising a thrombolytic agent.

5. Use of the compound(s) as defined in claims 1 to 4 for the manufacture of a medicament for preventing or treating a stroke, a myocardial infarction or a degenerative disorder, particularly Alzheimer's disease, Parkinson's disease and spinal injury.

**Revendications**

1. Médicament, contenant un ou plusieurs composés, qui inhibent l'action biologique de TNF$\alpha$ et CD95L, choisi parmi

- un anticorps anti-TNFα neutralisant et un anticorps anti-CD95L neutralisant ou des fragments de ceux-ci, ou
- un récepteur TNFα soluble et un récepteur CD95 soluble ou des portions de ceux-ci.

2. Médicament selon la revendication 1, dans lequel l'anticorps est un anticorps monoclonal ou un fragment de celui-ci.

3. Médicament selon la revendication 2, dans lequel l'anticorps monoclonal est un anticorps provenant d'un animal, un anticorps humanisé ou un anticorps chimère ou un fragment de ceux-ci.

4. Médicament selon l'une des revendications 1 à 3, contenant de plus un agent thrombolytique.

5. Utilisation des compositions définies dans les revendications 1 à 4 pour la préparation d'un médicament destiné à la prévention ou au traitement d'une attaque, d'un infarctus cardiaque ou d'une maladie dégénérative, en particulier la maladie d'Alzheimer, de Parkinson et un traumatisme de la moelle épinière.

FIGUR 1

**A**

**B**

**C**

Infarct frequency density

- 0 %

- 100 %

wt     gld

tnf⁻/⁻     gld/tnf⁻/⁻

FIGUR 2

FIGUR 3

**A**

**B**

FIGUR 4

FIGUR 5

**A**

**B**

**C**

FIGUR 6

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- DE 4412177

### In der Beschreibung aufgeführte Nicht-Patentliteratur

- *Dokument Clinical and Experimental Immunology,* 1999, vol. 118 (1), 108-114
- *Dokument Blood,* 1997, vol. 90 (2), 542-548
- *The Journal of Neuroscience,* Mai 1999, vol. 19 (10), 3809-3817
- **QUEEN et al.** *Proc. Natl. Acad. Sci. USA,* 1989, vol. 86, 10029
- **VERHOEYAN et al.** *Science,* 1988, vol. 239, 1534
- **DHEIN et al.** *Nature,* 1995, vol. 373, 438-441
- **ZEA LONGA et al.** *Stroke,* 1989, vol. 20, 84
- **CONNOLLY et al.** *Neurosurgery,* 1996, vol. 38, 523
- **SWANSON et al.** *J.Cereb.Blood Flow Metab.,* 1990, vol. 10, 290